# EUROPEAN PATENT SPECIFICATION

(11) **EP 3 788 954 B1**
(45) Date of publication and mention of the grant of the patent: **13.04.2022**
(21) Application number: 19195872.7
(22) Date of filing: 06.09.2019
(51) Int. Cl.: A61B 5/291, A61B 5/296, A61B 5/25

(54) **LAYER STRUCTURE WITH LOCAL RESERVOIR**
SCHICHTSTRUKTUR MIT LOKALEM RESERVOIR
STRUCTURE STRATIFIÉE DOTÉE D'UN RÉSERVOIR LOCAL

(43) Date of publication of application: 10.03.2021
(73) Proprietor: SmartMedics Sp. z o.o., 02-566 Warsaw (PL)
(72) Inventor: Wróblewski, Grzegorz, 05-520 Bielawa (PL); Maciejewski, Adrian, 00-384 Warsaw (PL); Koltowski, Lukasz, 03-704 Warsaw (PL)
(74) Representative: Fuchs Patentanwälte Partnerschaft mbB

(56) References cited:
- US-A- 4 444 194
- US-A1- 2006 155 181
- US-A1- 2019 167 192

## Description

### Field of the invention

The present invention relates to a layer structure for application to a surface of a subject, in particular for use in medical products such as a medical patch.

### Background of the invention

In both the medical and non-medical field, a wide range of layer structures are employed to interact with a surface of a subject, e.g. a body of a patient.

For example, layer structures in form of electrode patches are used for ECG monitoring (ECG: electrocardiography), i.e. in order to obtain biometric parameters of a subject. A prior art electrode patch is known from document US 2012/323104 A1.

Further, layer structures are also known from the cosmetic field, where layer structures are provided in the form of cosmetic or dermapharmaceutical patches. Moreover, layer structures can be for example be used for EMS-clothing (EMS: electrical muscle stimulation).

Document US 4,444,194 A discloses a medical electrode assembly for use in EKG testing procedures.

Document US 2006/155181 A1 discloses a measuring electrode arrangement for electroimpedance tomography.

In many prior art layer structures that are to be applied to a surface of a subject and that are to interact with the subject, the layer structure is provided with some gel or other electrochemical coupling agent for improving the functionality of the layer structure. For example, in electrode patches, electrodes are often covered or coated by a conductive gel before application of the electrode patch to the patient to achieve a sufficient signal detection. Moreover, cosmetic patches are often applied to a subject in a pre-moistened condition, wherein the moisture improves the interaction of the patch with the subject. However, common gels or electrochemical coupling agents are often insufficient, especially, for long term use. Further, pre-moistened layer structures often have a limited shelf-life, while application of additional gels to the layer structure before application of the layer structure to the subject is often insufficient with regard to an optimal amount of gel and the ease of applying the gel at a desired location on the layer structure.

### Object of the invention

It is an object of the present invention to provide a layer structure for application to a surface of a subject that overcomes the above drawbacks.

In particular, it is an object of the present invention to provide a layer structure having an improved shelf-life and enabling long-term use of the layer structure.

Moreover, it is a preferred object of the present invention to provide a layer structure that is easily and quickly attachable to a subject.

These objects are achieved by the subject matter of the independent claim. Optional embodiments and optional features are specified in the dependent claims and the following description.

### Summary of the invention

An aspect of the invention relates to a layer structure for application to a surface of a subject, preferably to skin of a subject, such as a patient. The layer structure can be a substantially flat layer structure, a patch structure, and/or a layer assembly. The layer structure is a multiple electrode patch structure. In particular, the layer structure can be used for or in medical products, such as a medical patch. However, layer structure according to the invention is not limited to medical applications, but can also be used in other fields such as cosmetics, etc. Preferable technical applications of the layer structure are electrotherapy applications, diagnostic applications, multiple electrode patches, sensor applications, multiple sensors patches, applications for performing electroencephalography (EEG), electromyography (EMG), electrocardiography (ECG), electroculography (EOG), microdefibrillation, defibrillation, etc., EMS-clothing (EMS: electrical muscle stimulation), external cardiac mapping, device navigation, such as pacemaker navigation (based on monitored electrical signals), compresses and/or contraception patches with active substances (transdermal patches).

The layer structure comprises a support layer and a backing layer that is removably attached to the support layer. The backing layer can be removably attached to the support layer by an adhesive. The adhesive can be an adhesive layer such as an adhesive film provided on the support layer and/or on the backing layer. The adhesive can be elastic (stretchable). The adhesive can be an inherent property of the support layer and/or the backing layer. The adhesive can be provided locally in certain areas of the support layer and/or the backing layer or can cover substantially the whole surface of the support layer and/or the backing layer. The adhesive can be printed, dispensed, sprayed or deposited by means of other methods. In embodiments in which the adhesive is provided at least on the support layer, the adhesive can serve to securely attach the layer structure to the surface of the subject. The adhesive can be skin friendly so as to avoid skin irritation and enhance the comfortability of the layer structure. In particular, the adhesive can be biocompatible. Preferably, the ratio of cell survival of cells in contact with the adhesive during cytotoxicity test can be greater than 0.7, preferably greater than 0.8, more preferably greater than 0.9, tested in line with DIN EN ISO 10933-10:2010.

The layer structure comprises at least one local reservoir provided at a predetermined position between the backing layer and the support layer. The layer structure further comprises a fluidic substance and a solid substance being arranged inside the at least one local reservoir and being in contact with one another, wherein the fluidic substance is configured to continuously condition the solid substance. In particular, the solid substance is arranged so that a first surface of the solid substance faces the support layer, while a second surface of the solid substance opposite the first surface faces the backing layer. The fluidic substance and the solid substance can be in direct contact with one another. Alternatively, an additional separating layer can be arranged between the fluidic substance and the solid substance, for example to reduce and/or select the area of the contact surface so as to particularly influence the conditioning of the solid substance by the fluidic substance.

In the sense of this invention, the expression "solid substance" includes solid component (such as a foam and/or textile and/or porous solid), gel, and/or gel composition, wherein the gel/gel composition has a viscosity of at least 230000 mPas, preferably at least 500000 mPas, more preferably at least 800000mPas, and wherein the viscosity of the gel/gel composition is higher than a viscosity of the liquid substance. With other words, the solid substance is not limited to only solid components, but can also describe high viscous gels/gel compositions, i.e. solid-like gels/gel compositions. In the sense of this invention, the expression "liquid substance" includes liquid, gas, fluid, gel and/or gel composition. Viscosity values specified herein can relate to viscosities of the substances at 23°C and can be measured according to ISO 3219:1993, for example with Brookfield RST CC touch rheometer.

The viscosity of the solid substance can be higher than the viscosity of the liquid substance. Preferably, a ratio between the viscosity of the solid substance and the viscosity of the liquid substance, i.e. viscosity_{solid}/viscosity_{liquid}, can be at least 10, preferably at least 250, more preferably at least 1000, still more preferably at least 10000, even more preferably at least 100000. The gel (which can form the liquid substance and/or the solid substance) can be a crosslinked sol. The gel (which can form the liquid substance and/or the solid substance) can be a colloidal suspension. The viscosity of the liquid substance can be at least 0.25 mPas, preferably at least 0.5 mPas, more preferably at least 1 mPas. The viscosity of the liquid substance can be 220000 mPas or less, preferably 70000 mPas or less, more preferably 10000 mPas or less. The viscosity of the liquid substance can be between 0.25 mPas and 220000 mPas, preferably between 0.5 mPas and 70000 mPas, more preferably between 1 mPas and 10000 mPas. The viscosity of the solid substance can be at least 230000 mPas, preferably at least 500000 mPas, more preferably at least 800000 mPas. The viscosity of the solid substance can be 1000000000 mPas or less, preferably 100000000 mPas or less, more preferably 10000000 mPas or less. The viscosity of the solid substance can be between 230000 mPas and 1000000000 mPas, preferably between 500000 mPas and 100000000 mPas, more preferably between 800000 mPas and 10000000 mPas. All viscosity values specified herein can relate to viscosities of the substances at 23°C and can be measured according to ISO 3219:1993, for example with Brookfield RST CC touch rheometer.

The provision of interacting solid and fluidic substances in the layer structure, in particular the continuous conditioning of the solid substance by the fluidic substance, increases the shelf-life of the layer structure as a required condition of the solid substance can be maintained during storage life of the layer structure. In addition, by means of the solid substance long-term use of the layer structure can be allowed for. This applies even more as the solid substance is continuously conditioned by the fluidic substance so as to maintain the properties and functionality of the solid substance.

As the fluidic substance and the solid substance are arranged inside the same local reservoir and are in contact with one another, the continuous conditioning takes place automatically, i.e. in a predetermined and/or in a self-working manner. With other words, the continuous conditioning does not need to be manually executed.

Furthermore, by arranging or pre-positioning the solid and fluidic substances in the local reservoir, the continuous conditioning is carried out locally and thus selectively. More precisely, the fluidic substance only conditions the assigned solid substance, without conditioning further portions or large areas of the layer structure. Also, by arranging or pre-positioning the solid and fluidic substances in the local reservoir, the layer structure is immediately ready for application to the surface of the subject after removing the backing layer, without necessarily requiring further preparations such as adding additional fluidic substances to the layer structure or the surface of the subject. This simplifies attachment of the layer structure to the surface of the subject and reduces the preparation time for the user.

The layer structure comprises a plurality of local reservoirs, each local reservoir accommodating at least one solid substance and at least one fluidic substance as described with regard to the at least one local reservoir. The layer structure can comprise at least two local reservoirs, preferably at least twelve local reservoirs, more preferably at least 15 local reservoirs, still more preferably at least 18 local reservoirs, even more preferably more than 100 local reservoirs.

The layer structure comprises a plurality of electrodes. Thus, the layer structure can for example be used as an electrode patch for a multiple-lead ECG. In particular, each local reservoir is provided in the area of an electrode. Hence, the number of local reservoirs can be equal the number of electrodes provided in the layer structure. The electrode can comprise silver or silverchloride.

The layer structure can be configured so that in a state of the layer structure in which the backing layer is removed from the support layer and the layer structure is in contact with the surface of the subject, the fluidic substance is configured to continuously and locally condition at least one area of the surface of the subject, more precisely at least the area of the surface of the subject that is in contact with the fluidic substance. Thus, the fluidic substance can continuously condition both the solid substance and the at least one area of the surface of the subject. Thus, the layer structure can operate or carry out its intended function immediately after application of the layer structure to the surface of the subject by means of the fluidic substance. In contrast, in many known layer structures the user needs to wait for a certain time until adjustment of required physio-chemical conditions before using the layer structure. For example, in EMS-clothing or some diagnostic applications the user has to wait until a perspiration film has developed between electrodes/sensors and the surface of the subject so as to allow correct and sufficient signal transmission.

In an embodiment of the layer structure, the fluidic substance can be configured to continuously keep the solid substance in a desired state, preferably to continuously moisturize or preserve moisture of the solid substance, and/or to continuously preserve a predetermined physio-chemical condition in the region of the at least one reservoir. Thus, the fluidic substance can be configured to continuously keep in a desired state, preferably to continuously moisturize or preserve moisture of, and/or to continuously preserve a predetermined physio-chemical condition inside the at least one reservoir and/or an area of the subject to which the layer structure is applied.

In an embodiment, at least a portion of the fluidic substance can be arranged between the solid substance and the backing layer. Arrangement of at least a portion of the fluidic substance between the solid substance and the backing layer means that the solid substance can be spaced from backing layer thus forming a cavity between the solid substance and the backing layer, wherein the cavity can accommodate at least a portion of the fluidic substance. Another portion or amount of the fluidic substance can be absorbed by the solid substance. The solid substance can have a substantially circular cross-sectional area.

In an embodiment, the solid substance can have a recess in which the liquid substance is at least partially accommodated. For example, the solid substance can be ring-shaped, having an inner recess or opening, in which the liquid substance is partially or fully arranged. The recess can be circular, rectangular, oval, etc. In an embodiment, in which only a portion of the fluidic substance is arranged in the recess of the solid substance, another portion can be arranged between the solid substance and the backing layer. The recess can extend axially through the solid substance, i.e. can have the same thickness axial length as the axial length (thickness) of the solid substance. Alternatively, the recess can have an axial length, which is less than the thickness of the solid substance. In this case, e.g. a bottom portion of the solid component can be closed. For example, the bottom portion can be configured for contacting an electrode.

According to an embodiment, the fluidic substance can comprise a gaseous substance and/or a liquid substance, in particular a liquid gel, preferably a liquid hydrogel. The fluidic substance can be deposited in the at least one reservoir by dispensing, dosing, printing, pipetting, casting, etc.

According to an embodiment, the solid substance can comprise a solid gel, preferably a solid hydrogel, a foam and/or a fabric, preferably a non-woven fabric. Alternatively or in addition, the solid substance can comprise a semiconductor, a sensor component and/or a sensor surface. In particular, the solid substance can have certain, advantageous characteristics and/or can only operate depending on specific conditions of the solid substance. The condition of the solid substance can be dependent on, e.g. circumferential conditions, and can be particularly influenced by means of the fluidic substance.

In an embodiment of the layer structure, the fluidic substance can have adhesive and/or conductive properties. In particular, the fluidic substance can have adhesive and/or conductive properties at usual circumferential temperatures, e.g. in the range of 0 °C to at least 40 °C, preferably 5 °C to 35 °C, more preferably 10 °C to 30 °C. The conductivity of the fluidic substance can be at least 5,5·10⁻⁶ S/m, preferably at least 5·10⁻³ S/m. The conductivity of the fluidic substance can be less than 3·10² S/m, preferably less than 5 S/m. The conductivity of the fluidic substance can be in the range from 5,5·10⁻⁶ S/m to 3·10² S/m, preferably from 5·10-3 S/m to 5 S/m. Such a conductivity can be preferable for optimal signal transmission in case of using the layer structure in connection with electric components.

According to an embodiment, the backing layer and/or the support layer can comprise a pre-formed bulge portion. The pre-formed bulge portion can partially define the at least one local reservoir. The bulge portion can also be defined as a cup and/or pocket portion. When pre-formed in the backing layer, the bulge portion can be curved (convexed) in a direction facing away from the support layer. When pre-formed in the support layer, the bulge portion can be curved (convexed) in a direction facing away from the backing layer. The bulge portion can have a substantially circular cross-sectional area. The bulge portion can be dome-shaped.

In embodiments of the layer structure, which do not comprise any pre-formed bulge portion, the at least one local reservoir can be formed by a flexible and/or elastic (stretchable) deformation of the backing layer and/or the support layer by means of the fluidic substance deposited at a predetermined location of the layer structure between the backing layer and the support layer.

In an embodiment, the layer structure can comprise a protrusion that laterally borders the at least one local reservoir so as to prevent lateral leakage of the fluidic substance from the at least one local reservoir. The protrusion can laterally encircle the at least one reservoir. In embodiments with a plurality of local reservoirs, some or all of the reservoirs can be provided with a corresponding bordering protrusion.

Preferably, the protrusion is formed on the support layer and/or on the backing layer. The protrusion can be integral to the support layer and/or the backing layer, respectively. Alternatively, the protrusion can be a separate component, such as an additional layer. The protrusion can be printed, dispensed, sprayed or deposited by means of other methods.

The protrusion can have a substantially circular shape. The outer contour of the protrusion can be fully closed. The protrusion can be a full circle or can have an oval or rectangular shape.

In an embodiment, the layer structure can comprise at least one sensor component and/or actuator component arranged in the region of the at least one local reservoir between the solid substance and the support layer. The sensor component and/or the actuator component can be configured to interact with the surface of the subject via the solid substance and the fluidic substance.

According to an embodiment, the backing layer can comprise a polymer film, a paper film and/or a hydrogel film. The backing layer can be siliconized. The backing layer can be siliconized on a surface facing towards the support layer. In particular, the backing layer can be siliconized at a certain contact area of the backing layer that is in contact with the fluidic substance. Siliconizing the backing layer at least partially can be preferable for simple removability of the backing layer.

In an embodiment, the layer structure can comprise an absorption material that is attached to the backing layer at the area of the at least one reservoir. The absorption material can be configured to absorb and remove a partial amount of the fluidic substance upon removal of the backing layer. In particular, the absorption material can be configured to absorb and remove an amount of the fluidic substance that would be superfluous in a state in which the layer structure is applied to the surface of the subject. The absorption material can be attached to the backing layer on an inside of the bulge. The absorption material can be fabric, foam, non-woven, etc.

In an embodiment, the layer structure, in particular the backing layer and/or the support layer, can be provided with a barrier layer at the area of the at least one reservoir so as to prevent the fluidic substance from drying or dehumidifying. The barrier layer can for example be provided between the solid or fluidic substance and the backing layer or support layer. The barrier layer can be printed, spray-coated, coated or dispensed onto the backing layer and/or the support layer. The barrier layer can be a polymer layer, such as polymethyl methacrylate (PMMA) or thermoplastic polyurethane layer, preferably a polymer layer that is thermally or UV curable. The backing layer and the support layer can be provided with barrier layers comprising the same or different materials.

The barrier layer can be dielectric. The barrier layer can be arranged adjacent and preferably in direct contact with an electrode provided in the layer structure. The barrier layer can be provided between an electrode and the support layer.

The barrier layer can decrease the moisture vapor transmission rate (MVTR) of the layer structure in the area of the barrier layer (i.e. the area of the local reservoir) to 500 g/m² or less for 24 hours, preferably to 50 g/m² or less, more preferably to 5 g/m² or less.

The support layer can comprise polymer, such as a thermoplastic polymer, in particular at least one of TPU, PET, silicone, etc. Alternatively or additionally, the support layer can comprise other materials such as papers or textiles. The support layer can be non-woven, woven, a film, a foam, a coating, etc. The support layer can be siliconized. The support layer can be breathable. The support layer can have a moisture vapor transmission rate (MVTR) of at least 600 g/m² for 24 hours, preferably at least 700 g/m². The support layer can have a moisture vapor transmission rate from 700 to 25 000 g/m² for 24 hours.

The backing layer can comprise polymer, such as a thermoplastic polymer, in particular at least one of TPU, PET, silicone, etc. Alternatively or additionally, the backing layer can comprise other materials such as papers or textiles. The backing layer can be non-woven, woven, a film, a foam, a coating, etc. The backing layer can be siliconized. The backing layer can have a moisture vapor transmission rate (MVTR) of less than 5 g/m² for 24 hours, preferably less than 3.5 g/m², more preferably of 2 g/m² or less.

According to an embodiment, the fluidic substance can have a volume between 0.001 ml and 10 ml, preferably between 0.01 ml and 5 ml, more preferably between 0.1 ml and 2.5 ml, still more preferably between 0.5 ml and 1.5 ml. The fluidic substance can have a volume of at least 0.001 ml, preferably at least 0.01 ml, more preferably at least 0.1 ml, still more preferably at least 0.5 ml. The fluidic substance can have a volume of 10 ml or less, preferably 5 ml or less, more preferably 2.5 ml or less, still more preferably 1.5 ml or less.

According to an embodiment, the solid substance can have a thickness (axial extension) between 0.0024 mm and 2.4 mm, preferably between 0.01 mm and 2 mm, more preferably between 0.05 mm and 1.0 mm. The solid substance can have a thickness of at least 0.0024 mm, preferably at least 0.01 mm, more preferably 0.05 mm. The solid substance can have a thickness of 2.4 mm or less, preferably 2 mm or less, more preferably 1 mm or less.

In an embodiment, the impedance of the fluidic substance and/or the solid substance can be 3000 Ohms or less at 10Hz frequency, preferably 2900 Ohms or less, more preferably 2800 Ohms or less.

In an embodiment, the fluidic substance can have a volume resistivity in the range from 0.01 Ωm to 100 Ωm, preferably from 0.1 Ωm to 50 Ωm, more preferably from 1 Ωm to 10 Ωm. The fluidic substance can have a volume resistivity of at least 0.01 Ωm, preferably at least 0.1 Ωm, more preferably at least 1 Ωm. The fluidic substance can have a volume resistivity of 100 Ωm or less, preferably 50 Ωm or less, more preferably 10 Ωm or less.

In an embodiment, the fluidic substance can have an adhesion to volume resistivity ratio, i.e. a ratio of stainless steel adhesion (180° peel in g/in) to volume resistivity (in Ω·in) in the range from 0.5 to 1.5, preferably from 0.6 to 1.0. The fluidic substance can have an adhesion to volume resistivity ratio of at least 0.5, preferably at least 0.6. The fluidic substance can have an adhesion to volume resistivity of 1.5 or less, preferably 1.0 or less. Such a ratio is preferable to realize sufficient adhesion and optimal signal transmission, considering that both the adhesion to the solid substance and to the surface of the subject and the volume resistivity influence the signal transmission.

The fluidic substance can have disinfecting properties and can be configured to disinfect the surface area of the subject, which comes into contact with the fluidic substance. The fluidic substance can have absorbing properties and can be configured to absorb residues, substances, particles from the surface area of the subject, which comes into contact with the fluidic substance.

In an embodiment, the layer structure can be biocompatible and/or skin friendly so as to avoid skin irritation and enhance the comfortability of the layer structure. In particular, the fluidic substance can be biocompatible and/or skin friendly. Preferably, the ratio of cell survival of cells in contact with the layer structure and/or the fluidic substance during cytotoxicity testing can be greater than 0.7, preferably greater than 0.8, more preferably greater than 0.9, tested in line with PN EN ISO 10933-5:2009. The layer structure and/or the fluidic substance can have a cumulative irritation index in the range from 0 to 1.9, more preferably from 0 to 0.4, more preferably from 0 to 0.1, tested in line with PN-EN ISO 10993-10:2015-2. The layer structure and/or the fluidic substance can have a sensitizing effect (grade) in the range from 0 to 1, more preferably of 0, according to the ISO scale defined in PN-EN ISO 10993-10:2015-2.

According to an embodiment, at least 90% of the area of the layer structure can be radiolucent by means of radiolucency of each of the support layer, the solid substance and/or the fluidic substance. Preferably, at least 95% of the area of the layer structure can be radiolucent, more preferably at least 97%. Still more preferably 100% of the area, i.e. the complete area, of the layer structure can be radiolucent. As the layer structure can have a substantially flat shape or configuration, the area of the layer structure refers to a surface area of the layer structure.

The term radiolucent as used herein refers to materials or composite materials, which are substantially or fully transparent to electromagnetic, magnetic and/or electric field and/or radiation employed in common (medical) imaging procedures, such as X-ray or MRI, in a manner and degree similar to human soft tissue, e.g. muscle tissue. In particular, the layer structure can be radiolucent to electromagnetic, magnetic and/or electric field and/or to electromagnetic, magnetic and/or electric field radiation. In other words, the layer structure in this embodiment does not block electromagnetic radiation of X-ray or MRI but allows it to pass, thereby not interferingly emerging in X-ray or MRI pictures. Thus, the layer structure can be worn by a patient during X-ray and/or MRI treatment without negatively affecting the treatment. In particular, radiolucency can mean that the layer structure, i.e. the radiolucent material, does not darken an image of a common hospital X-ray (RTG) and/or of fluoroscopy and X-ray films taken during angiographies and/or other cardio/neuro/radiological procedures (diagnostic and/or therapeutic) more than 60% of image intensity. An exemplary system for common hospital X-ray can be Siemens Artis Zee, with exemplary settings of the lamp voltage from 40 kV to 70kV and lamp current from 8 mA to 12 mA. This maximum darkening should not be exceeded so that the practitioner is still able to assess and evaluate the image. In particular, the radiolucency can mean that the layer structure, i.e. the radiolucent material, does not darken an image of a common hospital X-ray (RTG) more than 50 % of image intensity, preferably not more than 40 %, more preferably not more than 30 %, still more preferably not more than 28 %, still more preferably not more than 18 %, still more preferably not more than 5 %. The less the image intensity is darkened by the layer structure, the better the obtained image can be assessed and evaluated by the practitioner. In other words, radiolucency preferably means that the layer structure, i.e. the radiolucent material, does not attenuate the radiation more than 40.0 µGy/min, preferably not more than 30.0 µGy/min, more preferably not more than 26.0 µGy/min, still more preferably not more than 23.0 µGy/min. In other words, radiolucency preferably means that during a procedure the ratio of dose of radiation attenuated by the layer structure, i.e. the radiolucent material, to overall dose of radiation radiated does not exceed 5 %, preferably does not exceed 3.5 %, more preferably does not exceed 2 %, still more preferably does not exceed 1.7 %, still more preferably does not exceed 1.5 %.

In further embodiments, the layer structure can comprise additional layers, films, coating and/or components.

Another aspect of the invention relates to a method of manufacturing a layer structure of the type described above.

A further aspect of the invention relates to a storing system comprising a packaging container, a plurality of conditioning beads and the layer structure of the type described above, wherein the plurality of conditioning beads and the layer structure are accommodated in the packaging container. The packaging container can be a bag, a box or the like. The conditioning beads can be humidifiers or humidity stabilization beads. The conditioning beads can be adapted to maintain a desired condition within the packaging container, e.g. to maintain a desired humidity within the packaging container by releasing moisture.

### Brief description of the drawings

For a better understanding of embodiments of the invention and to show how the same may be carried into effect, reference will now be made, purely by way of example, to the accompanying drawings in which like numerals designate corresponding elements or sections throughout.

In the accompanying drawings:
Fig. 1 shows a schematic cross sectional view of a first embodiment of the layer structure, displaying the layer structure configuration in the region of a local reservoir.
Fig. 2 shows a schematic cross sectional view of a second embodiment of the layer structure, displaying the layer structure configuration in the region of a local reservoir.
Fig. 3 shows a schematic cross sectional view of a third embodiment of the layer structure, displaying the layer structure configuration in the region of a local reservoir.

### Detailed description of the drawings

Various examples of embodiments of the present invention will be explained in more detail by virtue of the following embodiments illustrated in the figures and/or described below.

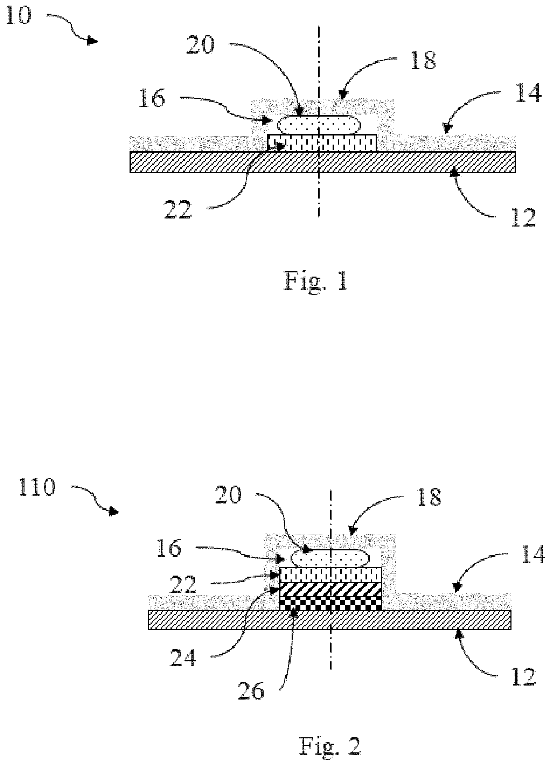

Figure 1 shows a layer structure 10 comprising a support layer 12 and a backing layer 14 that is removably attached to the support layer 12 by an adhesive film (not shown) provided on the support layer 12. In the state shown in Figure 1, the layer structure 10 can be stored or transported before use of the layer structure 10, i.e. before application of the layer structure 10 to a surface of a subject, such as to skin of a patient. In the shown embodiment, the support layer 12 is a breathable TPU layer and the backing layer 14 is a PET layer.

The layer structure 10 comprises a local reservoir 16 provided at a predetermined position between the backing layer 14 and the support layer 12. The local reservoir 16 is formed by a bulge 18 configured in a portion of the backing layer 14. The bulge 18 has a convex curvature in a direction facing away from the support layer 12 and is substantially dome-shaped.

A fluidic substance 20 and a solid substance 22 are arranged inside the local reservoir 16. In the shown embodiment, the fluidic substance 20 is a liquid hydrogel, while the solid substance 22 is a solid hydrogel. The fluidic substance 20 and the solid substance 22 are in direct contact with one another, wherein the fluidic substance 20 is positioned between the solid substance 22 and the backing layer 14. By means of the direct contact the fluidic substance 20 continuously moisturizes the solid substance 22. Thus, the fluidic substance 20 preserves a desired condition of the solid substance 22 and keeps the solid substance 20 in a desired state.

In a state in which the backing layer 14 is removed from the support layer 12, the rest of the layer structure 10 can be securely attached to the surface of the subject by means of the adhesive film provided on the support layer 12. With other words, when attached to the surface of the subject, the support layer 12 is at least partially in contact with the surface of the subject via the adhesive film. At the same time, the fluidic substance 20 is locally in direct contact with the surface of the subject. Locally means that the fluidic substance 20 is in contact with the surface of the subject at a selected position, predetermined by the location of the reservoir 16 on the layer structure 10 and by the positioning of the layer structure 10 on the surface of the subject. By selectively contacting the surface of the subject, the fluidic substance 16 also continuously conditions an area of the surface of the subject. Thus, in this embodiment, the fluidic substance 16 when in contact with the surface of the subject continuously moisturizes both the solid substance and the area of the surface of the subject at the same time. Thus, the layer structure 10 can carry out its intended function immediately after application of the layer structure 10 to the surface of the subject.

Figure 2 shows a layer structure 110 according to a second embodiment. Same or analog components and features are provided with the same reference signs as in Figure 1. In addition to the embodiment of the layer structure 10 shown in Figure 1, the layer structure 110 of Figure 2 further comprises a silver or silver chloride electrode 24, which is arranged subjacent the solid substance 22. Hence, the local reservoir 16 is arranged in the region of the electrode 24 and encompasses the electrode 24.

When the layer structure 10 (or at least a part of the layer structure 10) of Figure 2 is attached to the surface of the subject, in particular to skin of a patient, the electrode 24 is in operative connection with the surface of the subject via the fluidic substance 20 and solid substance 22. The fluidic substance 20 continuously conditions or moisturizes the solid substance 22 and the surface of the subject as described with regard to the embodiment of Figure 1. The combination of the fluidic substance 20 and the solid substance 22 and its interaction serves to achieve an optimal signal transmission between the surface of the subject and the electrode 24, which allows for a sufficient monitoring of a patient. Sufficient signal quality is provided immediately after application of the layer structure 10 to the surface of the subject by means of a pre-conditioned solid substance 22 and an immediate conditioning of the surface of the subject both by the fluidic substance 20.

The shown layer structure 110 further comprises a barrier layer 26 arranged subjacent to the electrode 24, between the electrode 24 and the support layer 12. The barrier layer 26 is dielectric and thus shields the electrode 24 in a direction towards the support layer 12. Further, the barrier layer 26 has a moisture vapor transmission rate (MVTR) of 500 g/m² or less for 24 hours, preferably to 50 g/m² or less, more preferably to 5 g/m² or less. Thus, the barrier layer 26 prevents or at least reduces drying of the components contained in the reservoir 16, i.e. of the fluidic substance 20 and the solid substance 22. This applies both for a state of storing/transporting the layer structure 10, i.e. with the backing layer 14 attached to the support layer 12, and to a state in which the layer structure 10 (without the backing layer 14) is attached to the surface of the subject. Thus, the barrier layer 26 increases both shelf-life of the layer structure 10 and long-term use of the layer structure 10.

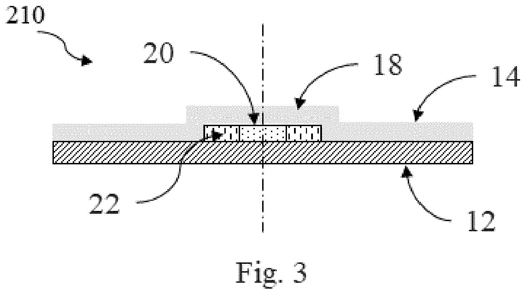

Figure 3 shows a layer structure 210 according to a third embodiment. Same or analog components and features are provided with the same reference signs as in Figures 1 and 2. In contrast to the first embodiment of Figure 1, the solid substance 22 of Figure 3 is ring-shaped and comprises an inner circular recess in which the liquid substance 20 is arranged. The inner circular recess extends completely through solid substance 22 in an axial direction. Thus, in this embodiment, both the solid substance 22 and the fluidic substance 20 can be in contact with an adjacent surface on top of the substances, i.e. a surface of a subject, and in contact with a subjacent component, e.g. an electrode (not shown in Fig. 3).

## Claims

1. Layer structure (10, 110) for application to a surface of a subject, in particular for use in medical products, the layer structure (10, 110) forming a multiple electrode patch structure comprising
a support layer (12);
a backing layer (14) that is removably attached to the support layer (12);
a plurality of electrodes (24);
a plurality of local reservoirs (16) each provided at a predetermined position between the backing layer (14) and the support layer (12) in an area of one of the plurality of electrodes (24); and
a fluidic substance (20) and a solid substance (22) being arranged inside each of the plurality of local reservoirs (16) and being in contact with one another, and
wherein the fluidic substance (20) is configured to continuously condition the solid substance (22).

2. Layer structure (10, 110) according to claim 1, wherein in a state in which the backing layer (14) is removed from the support layer (12) and the layer structure (10, 110) is in contact with the surface of the subject, the fluidic substance (20) is configured to continuously and locally condition at least one area of the surface of the substrate.

3. Layer structure (10, 110) according to claim 1 or 2, wherein the fluidic substance (20) is configured to continuously keep the solid substance (22) in a desired state, preferably to continuously moisturize the solid substance (22) and/or to continuously preserve a predetermined physio-chemical condition in the region of the at least one reservoir (16).

4. Layer structure (10, 110) according to one of the preceding claims, wherein at least a portion of the fluidic substance (20) is arranged between the solid substance (22) and the backing layer (14).

5. Layer structure (10, 110) according to one of the preceding claims, wherein the fluidic substance (20) comprises a gaseous substance and/or a liquid substance, in particular a liquid gel, preferably a liquid hydrogel.

6. Layer structure (10, 110) according to one of the preceding claims, wherein the solid substance (22) comprises a solid gel, preferably a solid hydrogel, a foam and/or a fabric, preferably a non-woven fabric.

7. Layer structure (10, 110) according to one of the preceding claims, wherein the fluidic substance (20) has adhesive and/or conductive properties.

8. Layer structure (10, 110) according to one of the preceding claims, wherein the backing layer (14) and/or the support layer (12) comprises a pre-formed bulge portion (18), which partially defines the at least one local reservoir (16).

9. Layer structure (10, 110) according to one of the preceding claims, further comprising a protrusion that laterally borders the at least one local reservoir (16) so as to prevent lateral leakage of the fluidic substance (20) from the at least one local reservoir (16), wherein preferably the protrusion is formed on the support layer (12) and/or on the backing layer (14).

10. Layer structure (110) according to one of the preceding claims, further comprising a sensor component (24) and/or actuator component (24) arranged in the region of the at least one local reservoir (16) between the solid substance (22) and the support layer (12).

11. Layer structure (10, 110) according to one of the preceding claims, wherein the backing layer (14) comprises a polymer film, a paper film and/or a hydrogel film,
the backing layer (14) being preferably siliconized, in particular at a contact area of the backing layer (14) that is in contact with the fluidic substance (20).

12. Layer structure (10, 110) according to one of the preceding claims, wherein an absorption material is attached to the backing layer (14) at the area of the at least one reservoir (16), the absorption material being configured to absorb and remove a partial amount of the fluidic substance (20) upon removal of the backing layer (14).

13. Layer structure (110) according to one of the preceding claims, wherein the backing layer (14) and/or the support layer (12) is provided with a barrier layer (26) at the area of the at least one reservoir (16) so as to prevent the fluidic substance (20) from drying, wherein preferably the barrier layer (26) is configured to decrease the moisture vapor transmission rate (MVTR) of the layer structure (10, 110) in the area of the barrier layer to 500 g/m² or less, preferably to 50 g/m² or less, more preferably to 5 g/m² or less

14. Layer structure (10, 110) according to one of the preceding claims,
wherein the fluidic substance (20) has a volume between 0.001 ml and 10 ml, preferably between 0.01 ml and 5 ml, more preferably between 0.1 ml and 2.5 ml, still more preferably between 0.5 ml and 1.5 ml; and/or
wherein the solid substance (22) has a thickness between 0.0024 mm and 2.4 mm, preferably between 0.01 mm and 2 mm, more preferably between 0.05 mm and 1.0 mm.

15. Layer structure (10, 110) according to one of the preceding claims, wherein the fluidic substance (20) has a volume resistivity in the range from 0.01 Ωm to 100 Ωm, preferably from 0.1 Ωm to 50 Ωm, more preferably from 1 Ωm to 10 Ωm; and/or
wherein the fluidic substance (20) has an adhesion to volume resistivity ratio in the range from 0.5 to 1.5, preferably from 0.6 to 1.0.

## Patentansprüche

1. Schichtstruktur (10, 110) zum Aufbringen auf eine Oberfläche eines Subjekts, insbesondere zur Verwendung in medizinischen Produkten, wobei die Schichtstruktur (10, 110) eine Mehrfachelektrodenpatch-Struktur bildet, mit:
einer Stützschicht (12);
einer abnehmbar an der Stützschicht (12) befestigten Trägerschicht (14);
einer Vielzahl von Elektroden (24);
einer Vielzahl von lokalen Reservoirs (16), die jeweils an einer vorgegebenen Position zwischen der Trägerschicht (14) und der Stützschicht (12) in einem Bereich einer der Vielzahl von Elektroden (24) vorgesehen sind; und
einer fluidischen Substanz (20) und einer festen Substanz (22), die innerhalb jedes der Vielzahl von lokalen Reservoirs (16) angeordnet sind und miteinander in Kontakt stehen,
wobei die fluidische Substanz (20) derart konfiguriert ist, dass sie die feste Substanz (22) kontinuierlich konditioniert.

2. Schichtstruktur (10, 110) nach Anspruch 1, wobei in einem Zustand, in dem die Trägerschicht (14) von der Stützschicht (12) entfernt ist und die Schichtstruktur (10, 110) in Kontakt mit der Oberfläche des Subjekts steht, die fluidische Substanz (20) dafür konfiguriert ist, mindestens einen Bereich der Oberfläche des Subjekts kontinuierlich und lokal zu konditionieren.

3. Schichtstruktur (10, 110) nach Anspruch 1 oder 2, wobei die fluidische Substanz (20) dafür konfiguriert ist, die feste Substanz (22) kontinuierlich in einem gewünschten Zustand zu halten, vorzugsweise die feste Substanz (22) kontinuierlich zu befeuchten und/oder kontinuierlich einen vorgegebenen physio-chemischen Zustand im Bereich des mindestens einen Reservoirs (16) aufrechtzuerhalten.

4. Schichtstruktur (10, 110) nach einem der vorangehenden Ansprüche, wobei mindestens ein Teil der fluidischen Substanz (20) zwischen der festen Substanz (22) und der Trägerschicht (14) angeordnet ist.

5. Schichtstruktur (10, 110) nach einem der vorangehenden Ansprüche, wobei die fluidische Substanz (20) eine gasförmige Substanz und/oder eine flüssige Substanz, insbesondere ein flüssiges Gel, vorzugsweise ein flüssiges Hydrogel, aufweist.

6. Schichtstruktur (10, 110) nach einem der vorangehenden Ansprüche, wobei die feste Substanz (22) ein festes Gel, vorzugsweise ein festes Hydrogel, einen Schaum und/oder einen Stoff, vorzugsweise einen Vliesstoff, aufweist.

7. Schichtstruktur (10, 110) nach einem der vorangehenden Ansprüche, wobei die fluidische Substanz (20) adhäsive und/oder leitfähige Eigenschaften aufweist.

8. Schichtstruktur (10, 110) nach einem der vorangehenden Ansprüche, wobei die Trägerschicht (14) und/oder die Stützschicht (12) einen vorgeformten Wölbungsabschnitt (18) aufweist, der das mindestens eine lokale Reservoir (16) teilweise definiert.

9. Schichtstruktur (10, 110) nach einem der vorangehenden Ansprüche, ferner mit einem Vorsprung, der das mindestens eine lokale Reservoir (16) seitlich begrenzt, um ein seitliches Austreten der fluidischen Substanz (20) aus dem mindestens einen lokalen Reservoir (16) zu verhindern, wobei der Vorsprung vorzugsweise auf der Stützschicht (12) und/oder auf der Trägerschicht (14) ausgebildet ist.

10. Schichtstruktur (110) nach einem der vorangehenden Ansprüche, ferner mit einer im Bereich des mindestens einen lokalen Reservoirs (16) zwischen der festen Substanz (22) und der Stützschicht (12) angeordneten Sensorkomponente (24) und/oder Aktuatorkomponente (24).

11. Schichtstruktur (10, 110) nach einem der vorangehenden Ansprüche, wobei die Trägerschicht (14) eine Polymerfolie, eine Papierfolie und/oder eine Hydrogelfolie aufweist, und
wobei die Trägerschicht (14) vorzugsweise silikonisiert ist, insbesondere an einem Kontaktbereich der Trägerschicht (14), der mit der fluidischen Substanz (20) in Kontakt steht.

12. Schichtstruktur (10, 110) nach einem der vorangehenden Ansprüche, wobei an der Trägerschicht (14) im Bereich des mindestens einen Reservoirs (16) ein Absorptionsmaterial befestigt ist, wobei das Absorptionsmaterial dafür konfiguriert ist, beim Entfernen der Trägerschicht (14) eine Teilmenge der fluidischen Substanz (20) zu absorbieren und zu entfernen.

13. Schichtstruktur (110) nach einem der vorangehenden Ansprüche, wobei die Trägerschicht (14) und/oder die Stützschicht (12) im Bereich des mindestens einen Reservoirs (16) eine Barriereschicht (26) aufweist, um ein Trocknen der fluidischen Substanz (20) zu verhindern, wobei die Barriereschicht (26) vorzugsweise dafür konfiguriert ist, die Wasserdampfdurchlässigkeit (MVTR) der Schichtstruktur (10, 110) im Bereich der Barriereschicht auf 500 g/m² oder weniger, vorzugsweise auf 50 g/m² oder weniger, besonders bevorzugt auf 5 g/m² oder weniger, zu vermindern.

14. Schichtstruktur (10, 110) nach einem der vorangehenden Ansprüche,
wobei die fluidische Substanz (20) ein Volumen zwischen 0,001 ml und 10 ml, vorzugsweise zwischen 0,01 ml und 5 ml, bevorzugter zwischen 0,1 ml und 2,5 ml, und noch bevorzugter zwischen 0,5 ml und 1,5 ml, aufweist, und/oder
wobei die feste Substanz (22) eine Dicke zwischen 0,0024 mm und 2,4 mm, vorzugsweise zwischen 0,01 mm und 2 mm, bevorzugter zwischen 0,05 mm und 1,0 mm, aufweist.

15. Schichtstruktur (10, 110) nach einem der vorangehenden Ansprüche,
wobei die fluidische Substanz (20) einen spezifischen Widerstand im Bereich von 0,01 Ωm bis 100 Ωm, vorzugsweise von 0,1 Ωm bis 50 Ωm, bevorzugter von 1 Ωm bis 10 Ωm, aufweist, und/oder
wobei die fluidische Substanz (20) ein Verhältnis von Adhäsion zu spezifischen Widerstand im Bereich von 0,5 bis 1,5, vorzugsweise von 0,6 bis 1,0, aufweist.

## Revendications

1. Structure stratifiée (10, 110) pour application sur une surface d'un sujet, en particulier pour une utilisation dans des produits médicaux, la Structure stratifiée (10, 110) formant une structure de patch à électrodes multiples comprenant
une couche de support (12) ;
une couche de soutien (14) qui est fixée de manière amovible à la couche de support (12) ;
une pluralité d'électrodes (24) ;
une pluralité de réservoirs locaux (16) prévus chacun à une position prédéterminée entre la couche de soutien (14) et la couche de support (12) dans une zone de l'une de la pluralité d'électrodes (24) ;
et
une substance fluidique (20) et une substance solide (22) étant disposées à l'intérieur de chacun de la pluralité de réservoirs locaux (16) et étant en contact l'une avec l'autre, et
dans lequel la substance fluidique (20) est configurée pour conditionner en continu la substance solide (22).

2. Structure stratifiée (10, 110) selon la revendication 1, dans laquelle dans un état dans lequel la couche de soutien (14) est retirée de la couche de support (12) et la structure stratifiée (10, 110) est en contact avec la surface du sujet, la substance fluidique (20) est configurée pour conditionner en continu et localement au moins une zone de la surface du substrat.

3. Structure stratifiée (10, 110) selon la revendication 1 ou 2, dans laquelle la substance fluidique (20) est configurée pour maintenir en continu la substance solide (22) dans un état souhaité, de préférence pour hydrater en continu la substance solide (22) et/ou pour préserver en continu un état physiochimique prédéterminé dans la région du au moins un réservoir (16).

4. Structure stratifiée (10, 110) selon l'une des revendications précédentes, dans laquelle au moins une partie de la substance fluidique (20) est disposée entre la substance solide (22) et la couche de soutien (14).

5. Structure stratifiée (10, 110) selon l'une des revendications précédentes, dans laquelle la substance fluidique (20) comprend une substance gazeuse et/ou une substance liquide, notamment un gel liquide, de préférence un hydrogel liquide.

6. Structure stratifiée (10, 110) selon l'une des revendications précédentes, dans laquelle la substance solide (22) comprend un gel solide, de préférence un hydrogel solide, une mousse et/ou un tissu, de préférence un tissu non tissé.

7. Structure stratifiée (10, 110) selon l'une des revendications précédentes, dans laquelle la substance fluidique (20) présente des propriétés adhésives et/ou conductrices.

8. Structure stratifiée (10, 110) selon l'une des revendications précédentes, dans laquelle la couche de soutien (14) et/ou la couche de support (12) comprend une partie bombée préformée (18), qui définit partiellement le au moins un réservoir local (16).

9. Structure stratifiée (10, 110) selon l'une des revendications précédentes, comprenant en outre une saillie qui borde latéralement l'au moins un réservoir local (16) de manière à empêcher une fuite latérale de la substance fluidique (20) de l'au moins un réservoir local (16), dans laquelle de préférence la saillie est formée sur la couche de support (12) et/ou sur la couche de soutien (14).

10. Structure stratifiée (110) selon l'une des revendications précédentes, comprenant en outre un composant capteur (24) et/ou un composant actionneur (24) disposé dans la région de l'au moins un réservoir local (16) entre la substance solide (22) et la couche de support (12).

11. Structure stratifiée (10, 110) selon l'une des revendications précédentes, dans laquelle la couche de soutien (14) comprend un film polymère, un film papier et/ou un film hydrogel,
la couche de soutien (14) étant de préférence siliconée, en particulier au niveau d'une zone de contact de la couche de soutien (14) qui est en contact avec la substance fluidique (20).

12. Structure stratifiée (10, 110) selon l'une des revendications précédentes, dans laquelle un matériau d'absorption est fixé à la couche de soutien (14) au niveau de l'au moins un réservoir (16), le matériau d'absorption étant configuré pour absorber et éliminer une quantité partielle de la substance fluidique (20) lors du retrait de la couche de soutien (14).

13. Structure stratifiée (110) selon l'une des revendications précédentes, dans laquelle la couche de soutien (14) et/ou la couche de support (12) sont pourvues d'une couche barrière (26) au niveau de l'au moins un réservoir (16) afin d'empêcher le séchage de la substance fluidique (20), dans laquelle de préférence la couche barrière (26) est configurée pour diminuer le taux de transmission de vapeur d'eau (MVTR) de la structure stratifiée (10, 110) dans la zone de la couche barrière à 500 g/m² ou moins, de préférence à 50 g/m² ou moins, plus préférablement à 5 g/m² ou moins.

14. Structure stratifiée (10, 110) selon l'une des revendications précédentes,
dans laquelle la substance fluide (20) a un volume compris entre 0,001 ml et 10 ml, de préférence entre 0,01 ml et 5 ml, plus préférablement entre 0,1 ml et 2,5 ml, encore plus préférablement entre 0,5 ml et 1,5 ml ; et/ou
dans laquelle la substance solide (22) a une épaisseur comprise entre 0,0024 mm et 2,4 mm, de préférence entre 0,01 mm et 2 mm, plus préférablement entre 0,05 mm et 1,0 mm.

15. Structure stratifiée (10, 110) selon l'une des revendications précédentes, dans laquelle la substance fluidique (20) présente une résistivité volumique comprise entre 0,01 Ωm et 100 Ωm, de préférence entre 0,1 Ωm et 50 Ωm, plus préférentiellement entre 1 Ωm et 10 Ωm ; et/ou
dans laquelle la substance fluidique (20) a un rapport entre l'adhérence et la résistivité volumique dans la gamme de 0,5 à 1,5, de préférence de 0,6 à 1,0.
